# EUROPEAN PATENT SPECIFICATION

(11) **EP 3 576 741 B1**
(45) Date of publication and mention of the grant of the patent: **29.07.2026**
(21) Application number: 17706311.2
(22) Date of filing: 02.02.2017
(51) Int. Cl.: A61K 31/4985, A61K 9/10, A61K 9/51, A61K 31/519, A61K 9/14

(54) **A METHOD OF MANUFACTURING A SUSPENSION OF NANOPARTICLES OF TADALAFIL OR SILDENAFIL CITRATE**
VERFAHREN ZUR HERSTELLUNG EINER SUSPENSION VON NANOPARTIKELN AUS TADALAFIL ODER SILDENAFILCITRAT
PROCÉDÉ DE FABRICATION D'UNE SUSPENSION DE NANOPARTICULES DE TADALAFIL OU DE CITRATE DE SILDÉNAFIL

(43) Date of publication of application: 11.12.2019
(73) Proprietor: Dukebox SP. Z O.O., 01-059 Warszawa (PL)
(72) Inventor: BANACH, Marcin, 32-100 Proszowice (PL); PULIT-PROCIAK, Jolanta, 31-866 Krakow (PL)
(74) Representative: Krekora, Magdalena
(86) International application number: PCT/IB2017/050575
(87) International publication number: WO 2018/142189

(56) References cited:
- WO-A1-2006/069419
- WO-A2-2007/033239
- US-A1- 2005 042 177
- US-A1- 2012 128 740
- US-A1- 2015 359 735

## Description

### TECHNICAL FIELD

A method of manufacturing a suspension of nanoparticles of tadalafil or sildenafil citrate.

### BACKGROUND ART

Sildenafil is a Phosphodiesterase 5 Inhibitor. Sildenafil functions as a selective and competitive inhibitor of type 5 phosphodiesterases (PDE5). PDE5 is a cyclic guanosine-3',5'-monophosphate (cGMP)-specific phosphodiesterase belonging to a class of phosphodiesterases which regulate various cell functions by catalyzing the hydrolysis of the second messenger molecules (cGMP) and cyclic adenosine-3',5'-monophosphate (cAMP). Sildenafil is used extensively for erectile dysfunction and less commonly for pulmonary hypertension. Sildenafil citrate has subsequently been commercially developed by Pfizer, Inc. and is available under the trademark VIAGRA.

Tadalafil is a carboline-based compound with vasodilatory activity. Tadalafil selectively inhibits the cyclic guanosine monophosphate (cGMP)-specific type 5 phosphodiesterase- (PDE-5)-mediated degradation of cGMP, which is found in the smooth muscle of the corpus cavernosa and corpus spongiosum of the penis. Inhibition of cGMP degradation by tadalafil results in prolonged muscle relaxation, vasodilation, and blood engorgement of the corpus cavemosa, and, so, prolonged penile erection. Tadalafil is used primarily to treat erectile dysfunction; benign prostatic hyperplasia and primary pulmonary hypertension. In USA tadalafil is approved as CIALIS for treating erectile dysfunction and ADCIRCA for the treatment of pulmonary arterial hypertension.

Patent application US2005042177A1 discloses nanoparticulate compositions comprising sildenafil free base. The sildenafil free base particles of the composition have an effective average particle size of less than about 2000 nm. The method of making this composition consists in dissolving the sildenafil free base particles in a solvent; adding the resulting sildenafil free base solution to a solution comprising at least one surface stabilizer; and precipitating the solubilized sildenafil free base having at least one surface stabilizer associated with the surface thereof by the addition thereto of a non-solvent. Stabilizers may be selected from a group consisting of *inter alia* gelatine, casein, lecithin (phosphatides), dextran, gum acacia, cholesterol, and stearic acid.

Patent application US2012128740 discloses sildenafil base or citric salts nanostructures having an average particle size of less than 500 nm, and a method of its manufacturing consisting of dissolving sildenafil base or its pharmaceutically acceptable salt or co-crystal and optionally one or more stabilizer or polyelectrolytes or a mixture thereof in a suitable solvent; adding the obtained formulation to a solution comprising one or more polyelectrolytes or stabilizers or a mixture thereof, if desired in the presence of a pharmaceutically acceptable acid or base; and precipitating the whole formulation.

Patent application HU1000214 discloses a method of manufacturing a sildenafil citrate nanoparticles composition in a microfluidic device. The method consists in dissolving sildenafil citrate in water, and adding Carbopol 980 dissolved in distilled water, where both solution are fed to the first microfluidic reactor unit. The resulting colloidal solution is fed to the second reactor unit where nanoparticles of sildenafil citrate are obtained. The nanoparticles size ranges from 70 nm to 500 nm.

Patent application WO2007033239A2 discloses compositions comprising nanoparticulate tadalafil, or a salt or derivative thereof. The nanoparticulate tadalafil particles of the composition have an effective average particle size of less than about 2000 nm and may be useful in the treatment of sexual dysfunction and vascular-, pulmonary- and cardiac-related diseases and conditions.

Patent application WO2007033239A1 discloses a method comprising dispersing particles of tadalafil, or a salt or derivative thereof, in a liquid dispersion medium, followed by subjecting the dispersion to homogenization to reduce the particle size of a tadalafil to the desired effective average particle size. The tadalafil particles can be reduced in size in the presence of at least one surface stabilizer. Alternatively, the tadalafil particles can be contacted with one or more surface stabilizers either before or after attrition. Other compounds, such as a diluent, can be added to the tadalafil/surface stabilizer composition either before, during, or after the size reduction process. Dispersions can be manufactured continuously or in a batch mode.

### DISCLOSURE OF THE INVENTION

The method in accordance with the present invention solves the problem of preparation of a stable suspension a suspension of nanoparticles of tadalafil or sildenafil citrate, and thus improving their bioavailability and efficacy.

A method of manufacturing a suspension of nanoparticles of tadalafil or sildenafil citrate of the present invention is characterised in that, a solution of tadalafil or sildenafil in dimethyl sulfoxide (DMSO) is prepared, where the concentration of sildenafil in DMSO is from 200 to 400 g/ dm³ or the concentration of tadalafil in DMSO is from 20 to 650 g/dm³, and an aqueous solution of a least one dispersing agent chosen from a group consisting of lecithin, gelatin and starch is prepared, then under continuous homogenization a solution of tadalafil or sildenafil is added portion-wise to the aqueous solution of at least one dispersing agent, afterwards homogenization is continued until a suspension of tadalafil or sildenafil citrate nanoparticles is obtained, and is homogenised through the use of ultrasonic frequencies from 1 to 90 minutes.

Preferably the dispersing agent is dissolved in water in temperature not higher than 90°C.

Preferably the aqueous solution of dispersing agents contains lecithin in concentration from 25.0 to 66.67 g/dm³ and gelatin in concentration from 3.33 to 20 g/dm³.

Preferably aqueous solution of dispersing agents contains lecithin in concentration from 25.0 to 66.67 g/dm³ and starch in concentration from 3.33 to 20 g/dm³.

Preferably the aqueous solution of dispersing agents contains lecithin in concentration from 25.0 to 66.67 g/dm³ and starch in concentration from 3.33 to 6.67 g/dm³, and gelatine in concentration from 3.33 to 6.67 g/dm³.

Preferably the aqueous solution of dispersing agents contains potassium sorbate. The potassium sorbate acts as a preservative.

Preferably the concentration of potassium sorbate is from 1,33 to 33,33 g/dm³.

Preferably the volume ratio of sildenafil citrate or tadalafil solution in DMSO to aqueous solution is from 0.1 to 0.4.

Preferably the strength of ultrasound is from 500 to 600 W.

Preferably the ultrasonication is carried out in flow system or in batch system.

The present disclosure refers also to a suspension of nanoparticles of tadalafil or sildenafil citrate nanoparticles obtained by method according to the invention.

A method of manufacturing a suspension of nanoparticles of tadalafil or sildenafil citrate of the present invention enables obtaining of dispersed nanoparticles of sildenafil citrate or tadalafil having average size from 50 to 700 nm. Obtained suspension is a final product, that may be administered to patients as a medicinal product, or may be used as a starting material for manufacture of medicinal products. The method is technologically very simple, safe and may be easily implemented on large scale production.

While the solution of tadalafil or sildenafil citrate is introduced into aqueous solution of other ingredients, the particles of tadalafil or sildenafil citrate are precipitated. Sonication process enables obtaining of particles having nanometric size. This size is preserved as a result of stabilisation activity of lecithin, gelatine and/or starch. Sonication may be carried out in a batch reactor or in a flow system, each time supplied with a sonication kit having appropriate power - preferably from 500 to 600 W.

### BEST MODE OF CARRYING OUT THE INVENTION

The following examples illustrate the invention.

### Example 1

A solution of sildenafil citrate was prepared by dissolving 20 g of sildenafil citrate in 50 cm³ of DMSO. A solution of dispersing agents was prepared by dissolving 10 g of lecithin and 2 g of gelatine in 150 cm³ of water in temperature 80°C. Under continuous homogenization conditions a solution of sildenafil citrate was added portion-wise to the dispersing agents solution. Afterwards homogenization was continued for 10 minutes. A homogeneous water suspension of sildenafil citrate was obtained comprising sildenafil citrate nanoparticles, having average size of 217 nm, and concentration of 100 mg/cm³.

After 48 hours the average size of nanoparticles was 162 nm. It confirms high stability of sildenafil citrate nanoparticles in the suspension.

### Example 2

A solution of sildenafil citrate was prepared by dissolving 40 g of sildenafil citrate in 100 cm³ of DMSO. A solution of dispersing agents and a preservative was prepared by dissolving 20 g of lecithin, 4 g of gelatine, and 1.6 g of potassium sorbate in 300 cm³ of water in temperature 80°C. Under continuous homogenization conditions a solution of sildenafil citrate was added portion-wise to the dispersing agents and preservative solution. Afterwards homogenization was continued for 15 minutes. A homogeneous suspension of sildenafil citrate was obtained comprising sildenafil citrate nanoparticles, having average size of 122 nm, and concentration of 100 mg/cm³.

Potential zeta was equal to -29 mV. The absolute value of the electrokinetic (zeta) potential confirms high stability of the nanoparticles suspension.

### Example 3

A solution of sildenafil citrate was prepared by dissolving 40 g of sildenafil citrate in 100 cm³ of DMSO. A solution of dispersing agents was prepared by dissolving 20 g of lecithin, 2 g of gelatine, and 2 g of starch in 300 cm³ of water in temperature 80°C. Under continuous homogenization conditions a solution of sildenafil citrate was added portion-wise to the dispersing agents solution. Afterwards homogenization was continued for 10 minutes. A homogeneous suspension of sildenafil citrate was obtained comprising 89% sildenafil citrate nanoparticles having average size of 390 nm, and 11% sildenafil citrate nanoparticles having average size of 110 nm. The concentration of sildenafil was 100 mg/cm³.

### Example 4

A solution of tadalafil was prepared by dissolving 40 g of tadalafil in 290 cm³ of DMSO. A solution of dispersing agents and a preservative was prepared by dissolving 100 g of lecithin, 20 g of gelatine, and 8 g of potassium sorbate in 1710 cm³ of water in temperature 80°C. Under continuous homogenization conditions a solution of tadalafil was added portion-wise to the dispersing agents and preservative solution. Afterwards homogenization was continued for 10 minutes. A homogeneous suspension of tadalafil was obtained comprising 64% tadalafil nanoparticles having average size of 326 nm, and 36% tadalafil nanoparticles having average size of 79 nm. The concentration of tadalafil was 20 mg/cm³.

### Example 5

A solution of tadalafil was prepared by dissolving 20 g of tadalafil in 50 cm³ of DMSO. A solution of dispersing agents and a preservative was prepared by dissolving 10 g of lecithin, 2 g of gelatine, and 0.8 g of potassium sorbate in 150 cm³ of water in temperature 80°C. Under continuous homogenization conditions a solution of tadalafil was added portion-wise to the dispersing agents and preservative solution. Afterwards homogenization was continued for 90 minutes. A homogeneous suspension of tadalafil was obtained comprising tadalafil nanoparticles having average size of 459 nm. The concentration of tadalafil was 100 mg/cm³.

## Claims

1. A method of manufacturing a suspension of nanoparticles of tadalafil or sildenafil citrate, **characterised in that**, a solution of tadalafil or sildenafil in dimethyl sulfoxide (DMSO) is prepared, where the concentration of sildenafil in DMSO is from 200 to 400 g/dm³ or the concentration of tadalafil in DMSO is from 20 to 650 g/dm³, and an aqueous solution of a least one dispersing agent chosen from a group consisting of lecithin, gelatin and starch is prepared, then under continuous homogenization a solution of tadalafil or sildenafil is added portion-wise to the aqueous solution of at least one dispersing agent, afterwards homogenization is continued until a suspension of tadalafil or sildenafil citrate nanoparticles is obtained, and is homogenised through the use of ultrasonic frequencies from 1 to 90 minutes.

2. A method according to claim 1, **characterized in that**, the dispersing agent is dissolved in water in temperature not higher than 90°C.

3. A method according to claim 1 or 2, **characterized in that**, the aqueous solution of dispersing agents contains lecithin in concentration from 25.0 to 66.67 g/dm³ and gelatin in concentration from 3.33 to 20 g/dm³.

4. A method according to claim 1 or 2, **characterized in that**, the aqueous solution of dispersing agents contains lecithin in concentration from 25.0 to 66.67 g/dm³ and starch in concentration from 3.33 to 20 g/dm³.

5. A method according to claim 1 or 2, **characterized in that**, the aqueous solution of dispersing agents contains lecithin in concentration from 25.0 to 66.67 g/dm³ and starch in concentration from 3.33 to 6.67 g/dm³, and gelatine in concentration from 3.33 to 6.67 g/dm³.

6. A method according to any of the preceding claims, **characterized in that**, the aqueous solution of dispersing agents contains potassium sorbate.

7. A method according to claim 6, **characterized in that**, the concentration of potassium sorbate is from 1,33 to 33,33 g/dm³.

8. A method according to any of the preceding claims, **characterized in that**, the volume ratio of sildenafil or tadalafil solution in DMSO to aqueous solution is from 0.1 to 0.4.

9. A method according to any of the preceding claims, **characterized in that**, the strength of ultrasound is from 500 to 600 W.

10. A method according to any of the preceding claims any of the preceding claims, **characterized in that**, the ultrasonication is carried out in flow system or in batch system.

## Patentansprüche

1. Verfahren zur Herstellung einer Suspension von Nanopartikeln aus Tadalafil oder Sildenafilcitrat, **dadurch gekennzeichnet, dass** eine Lösung von Tadalafil oder Sildenafil in Dimethylsulfoxid (DMSO) hergestellt wird, wobei die Konzentration von Sildenafil in DMSO 200 bis 400 g/dm³ beträgt oder die Konzentration von Tadalafil in DMSO 20 bis 650 g/dm³ beträgt, und eine wässrige Lösung mindestens eines Dispergiermittels, ausgewählt aus einer Gruppe bestehend aus Lecithin, Gelatine und Stärke hergestellt wird, dann unter kontinuierlicher Homogenisierung eine Lösung von Tadalafil oder Sildenafil portionsweise zu der wässrigen Lösung von mindestens einem Dispergiermittel zugegeben wird, anschließend die Homogenisierung fortgesetzt wird, bis eine Suspension von Tadalafil- oder Sildenafilcitrat-Nanopartikeln erhalten wird, und diese unter Verwendung von Ultraschallfrequenzen 1 bis 90 Minuten lang homogenisiert wird.

2. Verfahren nach Anspruch 1, **dadurch gekennzeichnet, dass** das Dispergiermittel in Wasser bei einer Temperatur von nicht mehr als 90 °C gelöst wird.

3. Verfahren nach Anspruch 1 oder 2, **dadurch gekennzeichnet, dass** die wässrige Lösung der Dispergiermittel Lecithin in einer Konzentration von 25,0 bis 66,67 g/dm³ und Gelatine in einer Konzentration von 3,33 bis 20 g/dm³ enthält.

4. Verfahren nach Anspruch 1 oder 2, **dadurch gekennzeichnet, dass** die wässrige Lösung der Dispergiermittel Lecithin in einer Konzentration von 25,0 bis 66,67 g/dm³ und Stärke in einer Konzentration von 3,33 bis 20 g/dm³ enthält.

5. Verfahren nach Anspruch 1 oder 2, **dadurch gekennzeichnet, dass** die wässrige Lösung von Dispergiermitteln Lecithin in einer Konzentration von 25,0 bis 66,67 g/dm³ und Stärke in einer Konzentration von 3,33 bis 6,67 g/dm³ sowie Gelatine in einer Konzentration von 3,33 bis 6,67 g/dm³ enthält.

6. Verfahren nach einem der vorstehenden Ansprüche, **dadurch gekennzeichnet, dass** die wässrige Lösung von Dispergiermitteln Kaliumsorbat enthält.

7. Verfahren nach Anspruch 6, **dadurch gekennzeichnet, dass** die Konzentration an Kaliumsorbat 1,33 bis 33,33 g/dm³ beträgt.

8. Verfahren nach einem der vorstehenden Ansprüche, **dadurch gekennzeichnet, dass** das Volumenverhältnis der Sildenafil- oder Tadalafil-Lösung in DMSO zur wässrigen Lösung 0,1 bis 0,4 beträgt.

9. Verfahren nach einem der vorstehenden Ansprüche, **dadurch gekennzeichnet, dass** die Ultraschallleistung 500 bis 600 W beträgt.

10. Verfahren nach einem der vorstehenden Ansprüche, **dadurch gekennzeichnet, dass** die Ultraschallbehandlung in einem Durchflusssystem oder in einem Chargensystem durchgeführt wird.

## Revendications

1. Une méthode de fabrication d'une suspension de nanoparticules de tadalafil ou de citrate de sildénafil, **caractérisé en ce que**, une solution de tadalafil ou de sildénafil dans le diméthylsulfoxyde (DMSO) est préparée, où la concentration de sildénafil dans le DMSO est de 200 à 400 g/dm³ ou la concentration de tadalafil dans le DMSO est de 20 à 650 g/dm³, et une solution aqueuse d'au moins un agent dispersant choisi dans un groupe constitué de lécithine, de gélatine et d'amidon est préparée, puis, sous homogénéisation continue, une solution de tadalafil ou de sildénafil est ajoutée par portions à la solution aqueuse d'au moins un agent dispersant, après quoi l'homogénéisation est poursuivie jusqu'à obtention d'une suspension de nanoparticules de tadalafil ou de de citrate sildénafil, et est homogénéisée par utilisation de fréquences ultrasonores pendant de 1 à 90 minutes.

2. Une méthode selon la revendication 1, **caractérisé en ce que** l'agent dispersant est dissous dans de l'eau à une température ne dépassant pas 90 °C.

3. Une méthode selon la revendication 1 ou 2, **caractérisé en ce que** la solution aqueuse d'agents dispersants contient de la lécithine en concentration de 25,0 à 66,67 g/dm³ et de la gélatine en concentration de 3,33 à 20 g/dm³.

4. Une méthode selon la revendication 1 ou 2, **caractérisé en ce que** la solution aqueuse d'agents dispersants contient de la lécithine en concentration de 25,0 à 66,67 g/dm³ et de l'amidon en concentration de 3,33 à 20 g/dm³.

5. Une méthode selon la revendication 1 ou 2, **caractérisé en ce que** la solution aqueuse d'agents dispersants contient de la lécithine en concentration de 25,0 à 66,67 g/dm³ et de l'amidon en concentration de 3,33 à 6,67 g/dm³, ainsi que de la gélatine en concentration de 3,33 à 6,67 g/dm³.

6. Une méthode selon l'une quelconque des revendications précédentes, **caractérisé en ce que** la solution aqueuse d'agents dispersants contient du sorbate de potassium.

7. Une méthode selon la revendication 6, **caractérisé en ce que** la concentration en sorbate de potassium est de 1,33 à 33,33 g/dm³.

8. Une méthode selon l'une quelconque des revendications précédentes, **caractérisé en ce que** le rapport volumique de la solution de sildénafil ou de tadalafil dans le DMSO à la solution aqueuse est de 0,1 à 0,4.

9. Une méthode selon l'une quelconque des revendications précédentes, **caractérisé en ce que** la puissance des ultrasons est cde 500 à 600 W.

10. Une méthode selon l'une quelconque des revendications précédentes, **caractérisé en ce que** l'ultrasonication est réalisée dans un système en continu ou dans un système discontinu.
